# EUROPEAN PATENT APPLICATION

(11) **EP 4 681 637 A1**
(43) Date of publication of application: **21.01.2026**
(21) Application number: 24306210.6
(22) Date of filing: 18.07.2024
(51) Int. Cl.: A61B 5/1455, A61B 5/0295, A61B 5/024

(54) **PHOTOPLETHYSMOGRAPHY SENSOR**

(71) Applicant: Koninklijke Philips N.V., 5656 AG Eindhoven (NL)
(72) Inventor: PAULUSSEN, Elvira, 5656 Eindhoven (NL); CLEMENTE DA COSTA, Andre, 5656 Eindhoven (NL); SCHWENK, Marcus, 5656 Eindhoven (NL); RESENDE LINS, Victor, 5656 Eindhoven (NL); VUGTS, Rien, 5656 Eindhoven (NL); MOSS, Yvonne, 5656 Eindhoven (NL); CANNON, Ryan, 5656 Eindhoven (NL); THEIL, Olivier, 5656 Eindhoven (NL); GROEN, Steve, 5656 Eindhoven (NL)
(74) Representative: Philips Intellectual Property & Standards

(57) **Abstract**

Proposed concepts thus aim to provide schemes, solutions, concept, designs, methods and systems pertaining to measuring oxygen saturation of blood within bodily tissue. In particular, embodiments aim to provide a system for measuring oxygen saturation of blood within bodily tissue. This can be achieved by scattering some of the emitted optical radiation, such that at least some of the non-functional emitted optical radiation (e.g., that would not be directed towards tissue and/or into a optical radiation-absorbing cushion, for example) will be scattered in such a way that it will become functional optical radiation that contributes to the blood oxygen saturation measurement.

## Description

### FIELD OF THE INVENTION

The invention relates to the field of Photoplethysmography "PPG" sensors and more particularly to PPG sensor systems for measuring oxygen saturation.

### BACKGROUND OF THE INVENTION

A PPG measurement refers to the use of optical measurements to detect blood volume changes. PPG measurements may be used to determine the values of physiological parameters such as heart rate, respiration rate, and blood oxygen saturation (e.g. SpO₂). PPG sensors typically transmit optical radiation to a bodily tissue and subsequently detect this optical radiation after it has interacted with the bodily tissue.

A pulse oximeter sensor is a type of PPG sensor that is used to measure the oxygen saturation level in the blood of a subject. Blood oxygen saturation is typically derived from the ratio of pulse amplitudes of red and infrared PPG signals (e.g. approximately 660 nm and approximately 880 nm or 940 nm, respectively) using the "ratio of ratios" technique. This metric quantifies the difference in optical absorption in tissue between oxygen-bound and oxygen-unbound haemoglobin at red versus infrared wavelengths. PPG signals may be obtained from various locations in the anatomy, including in the finger, nose (e.g. nasal alar, or a nasal septum), or ear lobe of a subject. A measurement of blood oxygen saturation obtained from the nasal alar, or a nasal septum may be referred-to as an "SpOz level".

In pulse oximeter sensors, optical radiation (typically within portions of the red, and infrared region of the optical spectrum) is transmitted through the bodily tissue and is then detected by an optical detector such as a photodiode. The optical radiation that travels through the tissue can be categorized into functional optical radiation and non-functional optical radiation. Functional optical radiation is optical radiation that contains pulsatile information from blood volume change in the cardiac cycle. Non-functional optical radiation, also known as shunt optical radiation, does not contain pulsatile information and can introduce calibration artifacts in pulse oximeter sensors, if detected. To prevent non-functional optical radiation from reaching the optical detector of the sensor, in most transmissive SpO₂ sensors, a cushion material that encloses the skin (e.g., finger, nostril ear lobe, etc.) is made from an optically absorbing (e.g. black) material so as to absorb red/infrared optical radiation. The black cushion material also prevents potential functional optical radiation that is backscattered and/or absorbed by the black cushion material from contributing to the vital signs signal.

Furthermore, it is desired that a higher proportion of the detected functional optical radiation is optical radiation with a short optical transmissive path and a high modulation depth as this results in a high signal-to-noise ratio. If non-pulsatile tissue is part of the optical radiation path (e.g., fat, bone, ligaments, nails, etc.) then this will result in long optical radiation paths with low modulation depths, and so is unwanted.

### SUMMARY OF THE INVENTION

The invention is defined by the claims.

According to examples in accordance with an aspect of the invention, there is provided a PPG sensor system for measuring oxygen saturation of blood within bodily tissue.

The system comprises: a support structure configured, in use, to receive or to couple to bodily tissue; an optical source arrangement coupled to the support structure and configured to emit optical radiation; an optical detector arrangement coupled to the support structure and configured to detect optical radiation incident on the optical detector arrangement; and an optical scatterer coupled to the support structure and comprising a volume scattering material;
wherein, in use, the optical scatterer is configured to scatter at least a portion of the emitted optical radiation.

Proposed concepts thus aim to provide schemes, solutions, concepts, designs, methods and systems pertaining to measuring oxygen saturation of blood within bodily tissue. In particular, embodiments aim to provide a system for measuring oxygen saturation of blood within bodily tissue by scattering some of the emitted optical radiation, such that at least some of the non-functional emitted optical radiation (e.g., that would not be directed towards the optical detector and/or directed into a optical radiation-absorbing cushion) will be scattered in such a way that it will become functional optical radiation that contributes to the blood oxygen saturation measurement.

In other words, it is proposed that by scattering at least some of the emitted optical radiation, some non-functional optical radiation will be scattered into becoming functional optical radiation, i.e., optical radiation that both travels through pulsatile tissue and is detected by the optical detector arrangement, thus contributing to the PPG sensor system's readings. This scattering can happen either before optical radiation has entered the bodily tissue or after, or both - all of these options can contribute to potentially converting some non-functional optical radiation into functional optical radiation by scattering it and essentially redirecting the optical radiation from a non-functional direction to a functional direction. For example, if this scattering were to occur after the optical radiation has travelled through pulsatile tissue, the optical scatterer may scatter that optical radiation that would not have reached the optical detector arrangement (e.g., a photodiode) in such a way that the optical detector can then detect it. In another example, if this scattering were to occur before the optical radiation has travelled through any tissue, the optical scatterer may scatter optical radiation that would not have travelled through pulsatile tissue and/or would have travelled through pulsatile tissue but not in a direction towards the optical detector in such a way that the optical radiation can then travel through pulsatile tissue and be detected. For instance, optical radiation leaving the light source in sideways-direction, is laterally shifted by the volume scatterer and potentially directed towards skin, whereas it would otherwise have been absorbed or reflected or scattered back towards the optical source.

The inventors have realised that by including an optical scatterer in the PPG sensor system, a relatively cheap and simple solution to increasing the proportion of functional optical radiation is provided, thereby resulting in a PPG sensor system that can more accurately and/or reliably measure oxygen saturation of blood within bodily tissue. As a result of increasing the proportion of functional light, a signal-to-noise ratio and/or power efficiency of the PPG sensor is increased.

In some embodiments, the at least a portion of the emitted optical radiation may comprise optical radiation emitted from the optical source arrangement and directed outside of a predetermined target region. For example, the predetermined target region may be a target region on the surface of the received/ coupled tissue (e.g., skin), for example, which is desired to be irradiated for the purposes of effective PPG. Scattering optical radiation that is directed outside of this predetermined target region (e.g., shunt optical radiation) can thus help to convert some non-functional optical radiation to functional optical radiation by redirecting it, potentially towards the predetermined target region and/or the optical detector arrangement.

In some embodiments, the optical scatterer may be configured such that, in use, optical radiation emitted from the optical source arrangement and directed towards the predetermined target region is transmitted directly into the received/ coupled bodily tissue without being scattered by the optical scatterer. This may thus ensure that functional optical radiation remains functional optical radiation and is not needlessly scattered and potentially converted into non-functional optical radiation.

In some embodiments, the optical scatterer may be laterally offset relative to the optical source arrangement. This, for example, is one way to ensure that functional optical radiation remains functional optical radiation, as the optical scatterer will thus not interrupt a direct path between the optical source arrangement and the predetermined target region, however, if optical radiation is directed outside of the predetermined target region (i.e., to the sides) it might come into contact with the laterally-offset optical scatterer and thus potentially be directed back towards the predetermined target region and/or the optical detector arrangement. In other words, it 'recycles' the functional light. Furthermore, this may artificially enlarge the optical radiation source, having optical radiation be emitted from a wider footprint.

In some embodiments, the optical scatterer may at least partially surround the optical source arrangement. This may thus increase the proportion of non-functional optical radiation scattered and thus potentially converted into functional optical radiation, as well as artificially enlarging the optical radiation source.

In some embodiments, the optical scatterer may comprise an opening for directly transmitting optical radiation emitted from the optical source arrangement and directed towards the predetermined target region without being scattered by the optical scatterer. An opening (or in other words, a window) may directly couple optical radiation to the skin without needlessly scattering it.

In some embodiments, the optical scatterer may comprise a protruding border surrounding the opening, protruding, in use, towards the received/ coupled bodily tissue. The protruding border around the opening may provide an effective way to capture non-functional optical radiation which is directed away from the predetermined target region (i.e., to prevent optical radiation from escaping sideways) and to potentially scatter it towards the predetermined target region and/or the optical detector arrangement. Again, this may also help to artificially enlarge the optical radiation source.

In some embodiments, the protruding border may be 0.5 to 5 millimetres thick. This range may provide a balance between having sufficient volume to scatter optical radiation effectively and avoiding to significant a reduction in the intensity of the optical radiation.

A volume scattering material is defined as a material in which optical scattering occurs within the bulk material. By contrast, a surface scattering material is a material in which optical scattering occurs solely at the surface of the material. In some embodiments, the volume scattering material may comprise a material having scattering particles or voids embedded therein. The material may be a polymer or a ceramic, for example. Examples of suitable polymers include one or more of: thermoplastic polymers (e.g. a thermoplastic elastomer "TPE", a thermoplastic polyurethane "TPU", a thermoplastic fiber, a thermoplastic resin); thermosetting plastics (e.g. an epoxy, polyimides, a polyester resin, and so forth); engineering polymers; foams; and silicones. The optical scattering may be provided by scattering particles (e.g. Titanium oxide), or voids (e.g. air pockets) that are embedded in such materials. An example technique for incorporating scattering particles of Al₂O₃ into ceramics is disclosed in the document WO2006097876. The size of the particles or voids may be chosen so as to provide a high degree of optical scattering within a portion of the optical spectrum corresponding to the range of optical wavelengths emitted by the optical source. These may all be effective volume scattering materials.

In some embodiments, the scattering particles may comprise titanium oxide particles. These may be particularly effective scattering particles.

In some embodiments, the system may further comprise a second optical scatterer coupled to the support structure and comprising a second volume scattering material; wherein, in use, the second optical scatterer is configured to scatter at least a portion of the optical radiation after it has been transmitted through the received/ coupled bodily tissue, and optionally wherein the second optical scatterer at least partially surrounds the optical detector arrangement. This may further increase the amount of functional optical radiation that is directed towards the optical detector arrangement because light which has travelled through pulsatile tissue but which is directed away from the optical detector (and thus will not contribute to the PPG measurement) may be scattered towards the optical detector.

In some embodiments, the optical scatterer may at least partially surround the optical detector arrangement. This may allow optical radiation which has travelled through pulsatile tissue but which is directed away from the optical detector to potentially be scattered towards the optical detector, thereby 'recycling' the functional light. In other words, the volume scattering material recycles optical radiation escaping from the skin that fails to be detected by the optical detector. Instead of returning it to the skin, some of the optical radiation reaches the detector via the volume scatterer, thereby providing optical radiation with another opportunity to be detected.

In some embodiments, the system may further comprise an absorptive shell partially surrounding the optical scatterer. This may allow some optical radiation which is emerging from the scatterer in a non-functional direction to be absorbed rather than leaking out of the PPG system. For example, the absorptive shell may comprise a black cushion, which also provides comfort.

In some embodiments, the opening may have a similar or the same footprint as the optical source arrangement. This may allow all optical radiation emitted by the optical source arrangement and directed towards the predetermined target region (i.e., substantially normal) to be directly transmitted to the received/ coupled bodily tissue without being needlessly scattered.

According to another aspect of the invention, there is provided a pulse oximeter device comprise the PPG sensor system of any herein-described embodiment.

According to another aspect of the invention, there is provided a system for measuring oxygen saturation of blood within bodily tissue, the system comprising the pulse oximeter device of any herein-described embodiment; and a processing arrangement configured to: receive from the pulse oximeter an optical radiation detection signal describing optical radiation detected by the optical detector arrangement of the pulse oximeter; and process the optical radiation detection signal to determine an oxygen saturation value.

According to yet another aspect of the invention, there is provided a method for operating a PPG sensor system, the PPG sensor system comprising: a support structure configured, in use, to receive or to couple to bodily tissue; an optical source arrangement coupled to the support structure and configured to emit optical radiation; an optical detector arrangement coupled to the support structure and configured to detect optical radiation incident on the optical radiation detector arrangement; and an optical scatterer coupled to the support structure and comprising a volume scattering material; the method comprising: emitting optical radiation from the optical source arrangement; scattering at least a portion of the emitted optical radiation with the optical scatterer; and detecting at least a portion of the emitted optical radiation at the optical detector arrangement after it has been transmitted through at least a part of the received or the coupled bodily tissue.

In some embodiments the at least a portion of the emitted optical radiation may comprise optical radiation emitted from the optical source arrangement and directed outside of a predetermined target region.

According to another aspect of the invention, there is provided a computer program comprising code means for implementing the method of any herein disclosed method when said program is run on a processing system.

Thus, there may be proposed concepts for measuring oxygen saturation of blood within bodily tissue, and this may be done based on the inclusion of an optical scatterer in a PPG sensor system which is configured to scatter at least a portion of the emitted optical radiation.

These and other aspects of the invention will be apparent from and elucidated with reference to the embodiment(s) described hereinafter.

### BRIEF DESCRIPTION OF THE DRAWINGS

For a better understanding of the invention, and to show more clearly how it may be carried into effect, reference will now be made, by way of example only, to the accompanying drawings, in which:
Fig. 1 is a schematic diagram illustrating an example of various optical paths in and around a finger arranged between an optical source arrangement and an optical detector arrangement in a PPG sensor, in accordance with some aspects of the present disclosure;
Fig. 2 is a simplified block diagram of a PPG sensor system for measuring oxygen saturation of blood within bodily tissue according to a proposed embodiment;
Fig. 3 is an illustration of a cross-section of a PPG sensor system for measuring oxygen saturation of blood within bodily tissue according to a proposed embodiment;
Fig. 4 is a more detailed illustration of a cross-section of part of a PPG sensor system for measuring oxygen saturation of blood within bodily tissue according to a proposed embodiment;
Fig. 5 is a more detailed illustration of a cross-section of part of a PPG sensor system for measuring oxygen saturation of blood within bodily tissue according to a proposed embodiment;
Fig. 6 is a simplified block diagram of a PPG sensor system for measuring oxygen saturation of blood within bodily tissue according to a proposed embodiment;
Fig. 7 is a simplified block diagram of a pulse oximeter device according to a proposed embodiment;
Fig. 8 is a simplified block diagram of a system for measuring oxygen saturation of blood within bodily tissue according to a proposed embodiment;
Fig. 9 is a simplified flow diagram of a method for operating a PPG sensor system according to a proposed embodiment;
Fig. 10 illustrates an example of a computer within which one or more parts of an embodiment may be employed;

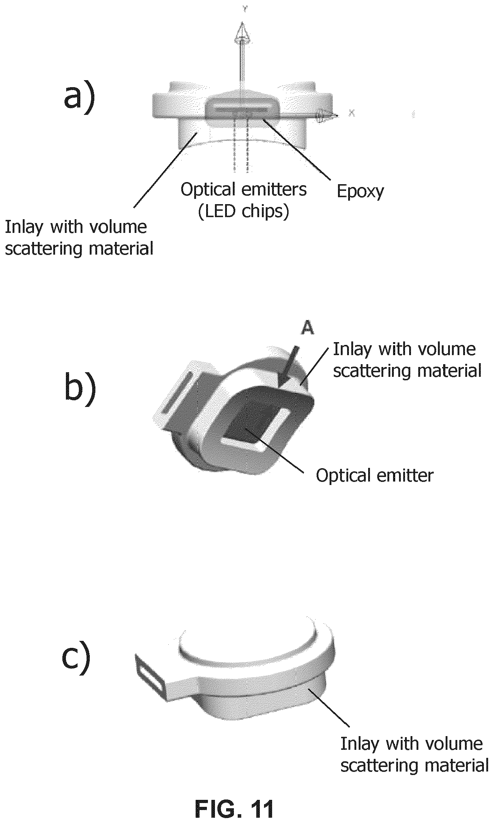

Fig. 11 is a schematic diagram illustrating three views a) - c) of an example of an optical source arrangement including a volume scattering material, in accordance with some aspects of the present disclosure;

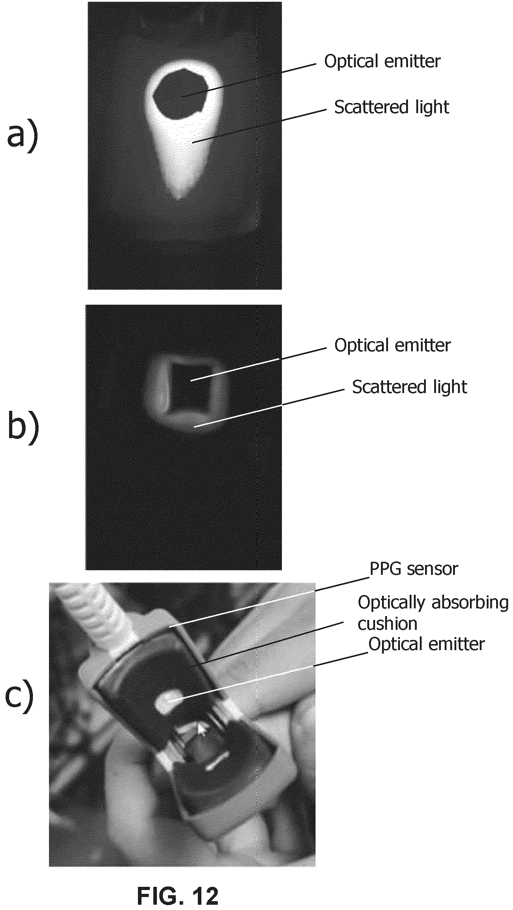

Fig. 12 is a schematic diagram illustrating the effect of volume scattering within a) an unconstrained border surrounding an optical source b) a spatially-constrained border surrounding an optical source c) a spatially-constrained border surrounding an optical source in a finger-based PPG sensor, in accordance with some aspects of the present disclosure;

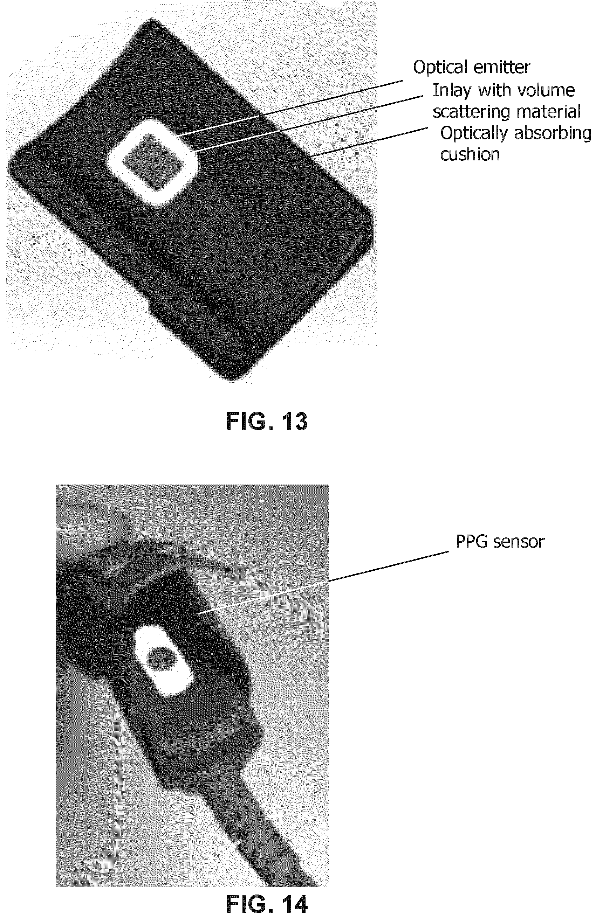

Fig. 13 illustrates an example of an inlay for a PPG sensor wherein the inlay includes an optical source, a spatially-constrained border surrounding the optical source wherein the spatially-constrained border is formed from a volume scattering material, and wherein the spatially-constrained border is surrounded by an optically absorbing cushion, in accordance with some aspects of the present disclosure;
Fig. 14 illustrates the inlay shown in Fig. 13 within a PPG sensor, in accordance with some aspects of the present disclosure;

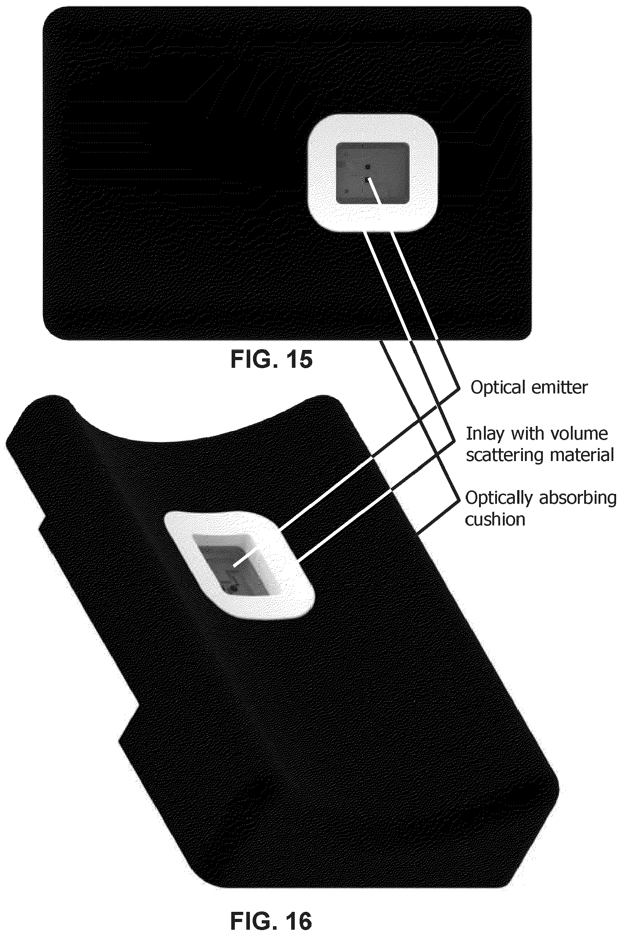

Fig. 15 illustrates a top view of an example of an inlay for a PPG sensor wherein the inlay includes an optical source, a spatially-constrained border surrounding the optical source wherein the spatially-constrained border is formed from a volume scattering material, and wherein the spatially-constrained border is surrounded by an optically absorbing cushion, in accordance with some aspects of the present disclosure;
Fig. 16 illustrates a first perspective view of the inlay illustrated in Fig. 15, in accordance with some aspects of the present disclosure.

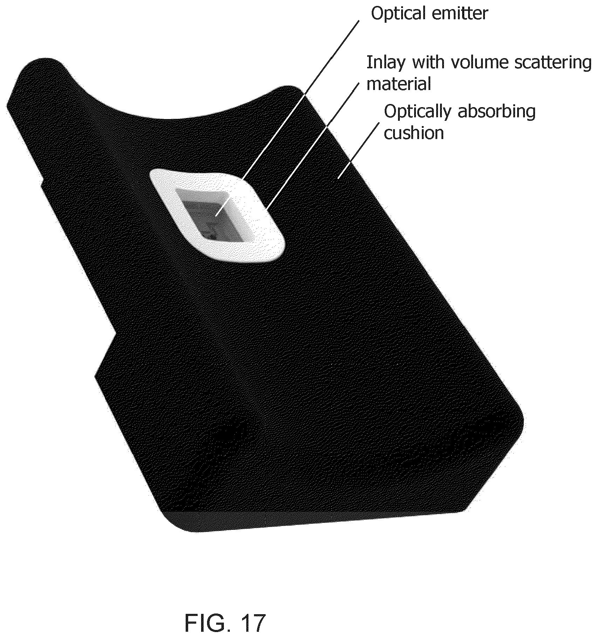

Fig. 17 illustrates a second perspective view of the inlay illustrated in Fig. 15, in accordance with some aspects of the present disclosure;

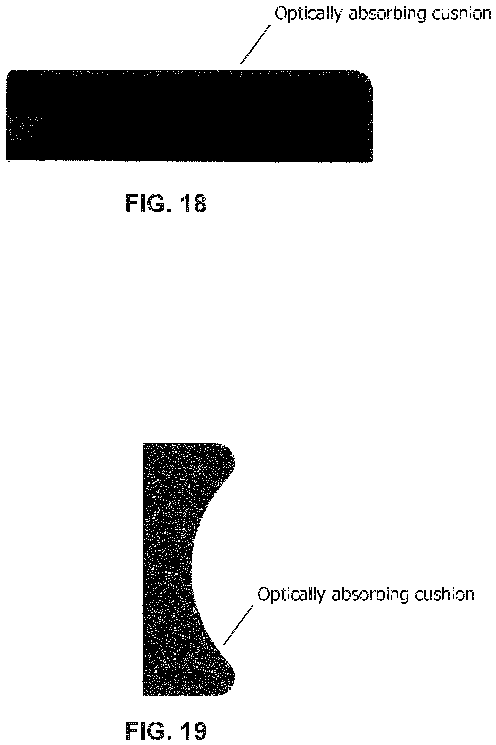

Fig. 18 illustrates a first side view of the inlay illustrated in Fig. 16, in accordance with some aspects of the present disclosure; and
Fig. 19 illustrates a second side view of the inlay illustrated in Fig. 15, in accordance with some aspects of the present disclosure.

### DETAILED DESCRIPTION OF THE EMBODIMENTS

The invention will be described with reference to the Figures.

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. It should be understood that the Figures are merely schematic and are not drawn to scale. It should also be understood that the same reference numerals are used throughout the Figures to indicate the same or similar parts.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality.

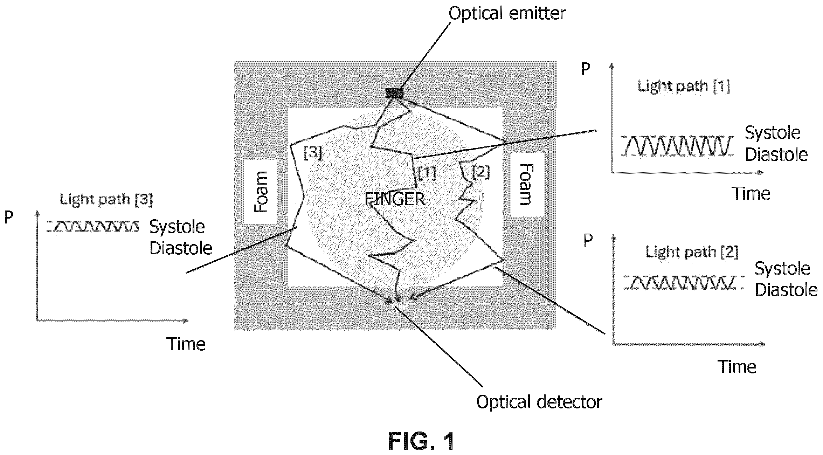

Fig. 1 is a schematic diagram illustrating an example of various optical paths in and around a finger arranged between an optical source arrangement and an optical detector arrangement in a PPG sensor, in accordance with some aspects of the present disclosure. In Fig. 1, the optical path [1] has a low transmission (`DC signal'), and high modulation depth (`AC signal'). The optical path [2] has an intermediate transmission (`DC signal') and intermediate modulation depth (`AC signal'). The optical path [3] has high transmission (`DC signal') and low modulation depth (`AC signal'). The total power detected at the optical detector is given by the sum of all ray paths contributing to the optical detector signal. The relative sizes of the modulation depths in detected optical signals for each of the paths [1] to [3] are illustrated in Fig. 1.

As may be seen from Fig. 1, short optical transmissive paths with large modulation depth are desired, resulting in high SNR. If non-pulsatile tissue is part of the optical light path (e.g. fat, bone, ligaments, nail), the consequence is long optical light paths with low modulation depths. Moreover, optical paths such as [2] and [3] in Fig. 1 should be avoided. Optical path lengths [2] and [3] occur if interaction with the surrounding sensor material takes place (reflection, transmission, scattering). In order to limit the optical paths [2] and [3] in Fig. 1, the surrounding material is typically made from a low reflectance, low transmittance (i.e. black) material. However, this causes the issue that the total detected optical signal in this case is low and total modulation depth is high. In order to have high SNR, both the total detected optical signal and the modulation depth should be high.

In order to address the foregoing, the present disclosure provides a PPG sensor system for measuring oxygen saturation of blood within bodily tissue.

Implementations in accordance with the present disclosure relate to various techniques, methods, schemes and/or solutions pertaining to measuring oxygen saturation of blood within bodily tissue. According to proposed concepts, a number of possible solutions may be implemented separately or jointly. That is, although these possible solutions may be described below separately, two or more of these possible solutions may be implemented in one combination or another.

Embodiments of the invention aim to provide a system for measuring oxygen saturation of blood within bodily tissue. This can be achieved by scattering some of the emitted optical radiation, such that at least some of the non-functional emitted optical radiation (e.g., that would not be directed towards a preferred portion of the received/ coupled tissue and/or would be directed into a optical radiation-absorbing cushion, for example) will be scattered in such a way that it will become functional optical radiation that contributes to the blood oxygen saturation measurement.

In other words, it is proposed that by scattering at least some of the emitted optical radiation, some non-functional optical radiation will be scattered into becoming functional optical radiation, i.e., optical radiation that will both travel through pulsatile tissue and be detected by the optical detector arrangement, thus contributing to the PPG sensor system's readings. This scattering can happen either before optical radiation has entered the bodily tissue or after, or both - all of these options can contribute to potentially converting some non-functional optical radiation into functional optical radiation by scattering it to essentially redirect the optical radiation from a non-functional direction to a functional direction.

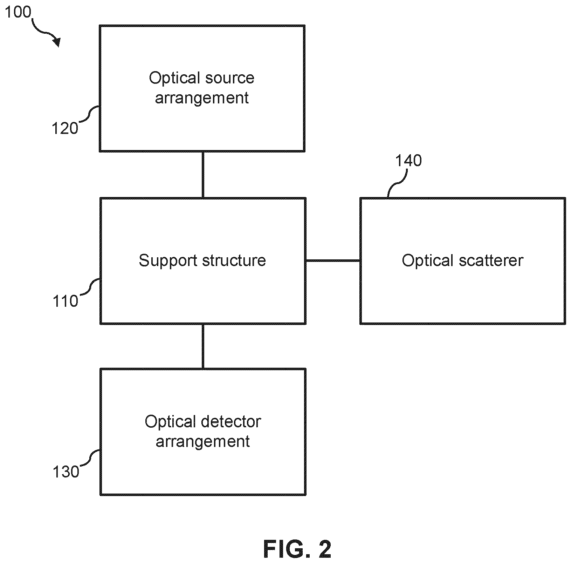

Referring now to Fig. 2, there is depicted a simplified block diagram of a PPG sensor system 100 for measuring oxygen saturation of blood within bodily tissue according to a proposed embodiment.

The system 100 comprises a support structure 110, an optical source arrangement 120, an optical detector arrangement 130, and an optical scatterer 140.

The support structure 110 is configured, in use, to receive or to couple to bodily tissue. As the skilled person would understand, the support structure 110 could take on a myriad of forms for this purpose, and its exact form is not important to the present invention. For example, it can take entirely typical forms, as would be known to the skilled person. The support structure may have the form of a sleeve, or a clip, for receiving the bodily tissue, for instance. The support structure may alternatively have the form of a surface for coupling to bodily tissue. The surface may form part of an inner surface of a limb-mounted (e.g. wrist-mounted) band, such as a watch, or an activity monitor, for example. The support structure 110 is merely for coupling the other components of the system 100 to one another and accommodating/ coupling to bodily tissue for irradiation by the optical source arrangement 120.

The optical source arrangement 120 is coupled to the support structure (e.g., either fixed or detachable) and configured to emit optical radiation. For example, for PPG, the optical radiation preferably comprises portions of the infrared and/or red optical spectrum. The optical source arrangement can comprise, for example, one or more LEDs (e.g., ceramic LED, lead frame LED, SMD LED, etc.), a laser diode, a super-luminescent diode, etc., or any other suitable optical source, as would be understood by the skilled person.

The optical detector arrangement 130 is coupled to the support structure (e.g., either fixed or detachable) and is configured to detect optical radiation incident on the optical detector arrangement. The optical detector arrangement can comprise, for example, a photodetector, a photodiode, a charge-coupled device, etc., or any other suitable optical detector, as would be understood by the skilled person.

The optical scatterer 140 is coupled to the support structure (e.g., either fixed or detachable) and comprises a volume scattering material. An optical scatterer 140 can also be referred to as a scattering shell or an optical scattering layer. The optical scatterer 140 can, for example, preferably be configured to scatter red optical radiation and/or infrared optical radiation. As mentioned above; a volume scattering material is defined as a material in which optical scattering occurs within the bulk material. By contrast, a surface scattering material is a material in which optical scattering occurs solely at the surface of the material. A volume scattering material may be a material that includes scattering particles or voids. The particles may have higher reflectivity than the surrounding material. In use (e.g., when the optical source arrangement is emitting optical radiation), the optical scatterer 140 is configured (e.g., it is positioned and shaped) to scatter at least a portion of the emitted optical radiation. In some embodiments, the optical scatterer 140 can consist of the volume scattering material, i.e., include no other components. In other embodiments, however, the optical scatterer 140 can include other components, such as, for example, a (highly) reflective surface layer. For example, the optical scatterer 140 can also be referred to as a (high) reflective inlay (e.g., an inlay in the support surface). Thus, the volume scatterer may be at least partially surrounded by a surface scatterer or specular reflector.

As mentioned above; a volume scattering material is defined as a material in which optical scattering occurs within the bulk material. By contrast, a surface scattering material is a material in which optical scattering occurs solely at the surface of the material. In some embodiments, the volume scattering material may comprise a material having scattering particles or voids embedded therein. The material may be a polymer or a ceramic, for example. Examples of suitable polymers include one or more of: thermoplastic polymers (e.g. a thermoplastic elastomer "TPE", a thermoplastic polyurethane "TPU", a thermoplastic fiber, a thermoplastic resin); thermosetting plastics (e.g. an epoxy, polyimides, a polyester resin, and so forth); engineering polymers; foams; and silicones. The optical scattering may be provided by scattering particles (e.g. Titanium oxide), or voids (e.g. air pockets) that are embedded in such materials. The size of the particles or voids may be chosen so as to provide a high degree of optical scattering within a portion of the optical spectrum corresponding to the range of optical wavelengths emitted by the optical source. These may all be effective volume scattering materials.

By way of an example, in one embodiment, the optical scatterer 140 could include titanium oxide particles embedded in a (biocompatible) silicone matrix.

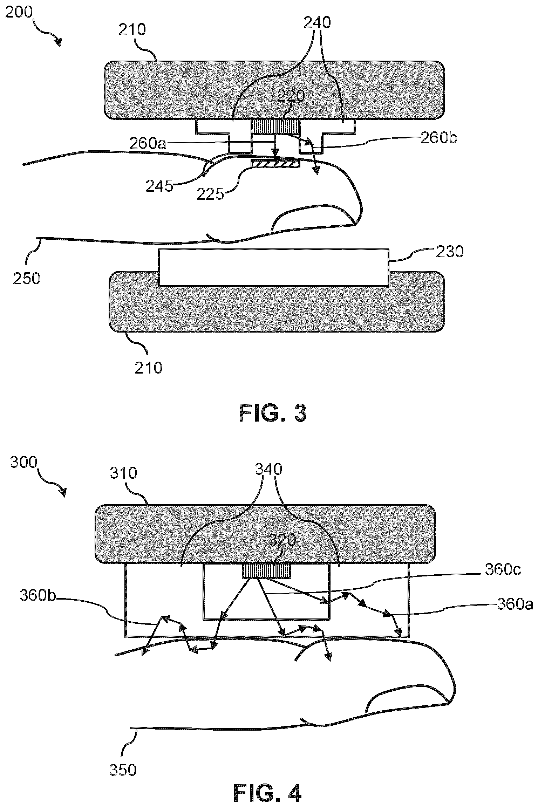

For example, in this embodiment, the at least a portion of the emitted optical radiation comprises optical radiation emitted from the optical source arrangement 120 and directed outside of a predetermined target region. For example, the predetermined target region can be a target (e.g., preferred) region on the surface of the received/ coupled tissue (e.g., skin), as exemplified in Fig. 3. The predetermined target region is a region that is desired to be irradiated for the purposes of effective PPG (i.e., a region on the surface of received/ coupled tissue, below which there is pulsatile tissue which is desired to be illuminated). Scattering optical radiation directed outside of this predetermined target region (i.e., shunt optical radiation) can thus help to convert some non-functional optical radiation to functional optical radiation by redirecting it, potentially towards the predetermined target region and/or the optical detector arrangement 130 (e.g., on the other side of the received tissue).

For example, in this embodiment, the optical scatterer 140 is further configured such that, in use, optical radiation emitted from the optical source arrangement 120 and directed towards the predetermined target region is transmitted directly into the received/ coupled bodily tissue without being scattered by the optical scatterer 140. This can thus ensure that functional optical radiation remains functional optical radiation and is not needlessly scattered and potentially converted into non-functional optical radiation.

To further this purpose, the optical scatter 140 can be laterally offset relative to the optical source arrangement 120. This, for example, is one way to ensure that functional optical radiation remains functional optical radiation, as the optical scatterer will thus not interrupt a direct path between the optical source arrangement and the predetermined target region, however, if optical radiation is directed outside of the predetermined target region (i.e., to the sides) it might come into contact with the laterally offset optical scatterer and thus potentially be directed back towards the predetermined target region and/or the optical detector arrangement 130. Furthermore, this can artificially enlarge the optical radiation source, having optical radiation be emitted from a wider footprint.

It should be noted that the optical scatterer 140 can be positioned in various ways with respect to the optical source arrangement 120 and/or the optical detector arrangement 130. For example, the optical scatterer 140 may at least partially surround the optical source arrangement 120 and/or the optical detector arrangement 140. The surrounding may for example be symmetrical, asymmetrical, or tailored to the emission of optical radiation from the optical radiation source, for example. This can thus increase the proportion of non-functional optical radiation scattered and thus potentially converted into functional optical radiation. It also has the effect of enlarging the optical radiation source/ detector. The optical scatterer may surround at least 50% of the surface of the optical source arrangement 120 and/or the optical detector arrangement 140, for example. In one embodiment, the optical scatterer 140 can fully surround (i.e. enclose) an optical radiation emitting region of the optical source arrangement 120 and/or the optical detector arrangement 130.

In one embodiment, the optical scatter 140 may include an opening for directly transmitting optical radiation emitted from the optical source arrangement and directed towards the predetermined target region without being scattered by the optical scatterer. An opening (i.e., a window) can provide an efficient method for directly transmitting functional optical radiation without needlessly scattering it. For instance, the opening can be a void, or the void may alternatively be filled with an optically-transparent material. The optically-transparent material may be a polymer (e.g. transparent silicone/ epoxy), for example. In some embodiments, there can also be an optical contact between the optical source arrangement 120 and the optically transparent material, as well as between the optically transparent material and the optical scatterer 140. This has the effect of reducing (Fresnel) reflection losses.

In an example, the opening of the optical scatterer 140 may have a similar, or the same, footprint as the optical source arrangement 120. The optical scatterer 140 having the same footprint as the optical radiation source can be understood as them sharing the same lateral dimensions (i.e., length and width, but not height), such that they occupy the same amount of space on the surface of the support structure 110, for example. This can allow substantially all optical radiation emitted by the optical source arrangement 120 and directed towards the predetermined target region (i.e., substantially normal) to be directly transmitted to the received/ coupled bodily tissue without being needlessly scattered.

It should be noted that in other embodiments, the optical scatter 140 can instead at least partially surround the optical detector arrangement 130. This can allow optical radiation which has travelled through pulsatile tissue but which is directed away from the optical detector to potentially be scattered towards the optical detector, thereby still helping to increase the amount of functional optical radiation which is detected and thus resulting in more accurate/reliable PPG measurements.

It should also be noted that in some embodiments, the system 100 can further comprise an absorptive shell partially surrounding the optical scatterer 140. This can allow some optical radiation which is emerging from the scatterer 140 in a non-functional direction to be absorbed rather than leaking out of the PPG sensor system 100. For example, the absorptive shell would not be positioned between the optical scatterer and the bodily tissue or between the optical scatterer and the predetermined target region. For example, the absorptive shell can comprise a black cushion, which also provides comfort. To be clear, by absorptive, it is meant that the shell comprises an optically absorptive material (e.g., absorbing at least red and/or infrared optical radiation). In one example, the shell is formed from a polymer. The shell may be formed from various polymers, such as those mentioned above.

It should also be noted, for completeness, that the present invention is suitable for use in both transmissive and reflective PPG sensors.

Referring now to Fig. 3, there is depicted an illustration of a cross-section of a PPG sensor system 200 for measuring oxygen saturation of blood within bodily tissue 250 according to a proposed embodiment.

The system 200 comprises a support structure 210 configured, in use (as illustrated) to receive or to couple to bodily tissue 250. In this instance, the bodily tissue 250 may include a portion of a finger, or a portion of the ear (lobe), or a portion of the nostril, for example. It should be noted that in this embodiment, the support structure 210 encircles the finger 250, forming a tube or pipe, but in other embodiments, as the skilled person would understand, this not need be the case, e.g., it may only partially surround the bodily tissue 250.

Coupled to the support structure 210 is an optical source arrangement 220, and in this embodiment the optical source arrangement 220 comprises one or more LEDs (preferably red and/or infrared). Coupled to the support structure 210, on the other side of the bodily tissue 250 (e.g., opposite the LEDs 220) is an optical detector arrangement 230. In this embodiment, the optical detector arrangement 230 comprises a optical detector. The optical detector arrangement 230 is configured to detect optical radiation incident on itself (e.g., after optical radiation emitted from the LEDs 220 has passed through the finger 250).

Also coupled to the support structure 210 is an optical scatterer 240 comprising a volume scattering material. As can be seen, in use, the optical scatter is configured (i.e., positioned and shaped) to scatter at least a portion of the optical radiation emitted from the optical source arrangement 220. One example of an emitted optical radiation ray scattered by the optical scatterer 240 is given by arrows 260b. This optical radiation ray 260b that was going to enter the bodily tissue 250 at such an angle that it would be unlikely to ever reach the optical detector 230 is instead scattered into entering the bodily tissue 250 at a more desirable angle such that it will more likely reach the optical detector 230, i.e., become functional optical radiation contributing to the PPG measurement.

In other words, optical radiation paths which are directed sideways of the LED 220 (particularly an issue with regular leadframe/SMD LEDs) and into the optical scatterer 240 can potentially contribute to the functional optical radiation as well as the virtual enlargement of the optical radiation source 220.

In this embodiment, the optical scatterer 240 at least partially surrounds the optical source arrangement 220 and is laterally offset relative to the optical source arrangement (i.e., no portion of the optical scatterer is disposed in a path between the optical source arrangement and the bodily tissue 250). This thus naturally leads to the optical scatterer 240 comprising an opening through which optical radiation emitted from the optical source arrangement 220 can be directly transmitted (e.g., passed through) towards a predetermined target region 225 without being scattered by the optical scatterer 240. An example of such a directly transmitted ray is given by arrow 260a.

As mentioned above, the predetermined target region 225 is a region that is desired to be irradiated for the purposes of effective PPG (i.e., a region on the surface of received/ coupled tissue 250, below which there is pulsatile tissue which is desired to be illuminated before the optical radiation reaches the optical detector 230). Scattering optical radiation directed outside of this predetermined target region 225 (i.e., shunt optical radiation) can thus help to convert some non-functional optical radiation to functional optical radiation by redirecting it, potentially towards the predetermined target region 225 and/or the optical detector arrangement 230 (e.g., on the other side of the received tissue and as shown by arrow 260b).

In this embodiment, the optical scatterer 240 comprises a protruding border 245 surrounding the opening. In this embodiment then, the optical scatterer 240 (including the protruding border 245) surrounds the optical source arrangement 220 on the inside surface of the support structure 210. In other embodiments, however, this need not be the case. In other embodiments, the optical scatterer 240 (and its protruding border 245, if present) may only flank the optical source arrangement 220 on a number of sides, for instance, two opposite sides, or only one side, or three sides, etc. The protruding border 245 protrudes, in use (as depicted in Fig. 3) towards the received/ coupled bodily tissue 250. The protruding border 245 around the opening provides an effective way to capture non-functional optical radiation which is directed away from the predetermined target region 225 (i.e., to prevent optical radiation from escaping sideways) and to potentially scatter it towards the predetermined target region 225 and/or the optical detector arrangement 230, as exemplified by optical radiation ray 260b. This can also help to artificially enlarge the optical radiation source in that optical radiation is emitted from not only the area of the optical source arrangement 220 but also from the tops of the protruding border 245 too, essentially making the optical radiation source wider (though the intensity of the optical radiation will obviously be lower emitting out of the optical scatterer 240 than the optical source arrangement 220). Of course, the skilled person would appreciate that the protruding border 245 is not essential to the working of the invention, for example, in other embodiments, the entire optical scatterer 240 can protrude towards the bodily tissue 250 relative to the optical source arrangement 220.

In this embodiment, the protruding border 245 is 0.5 to 5 millimetres thick. To be clear, that is the lateral thickness of the protruding border 245, parallel to the length of the support structure 210 or bodily tissue 250, i.e., not the height of the protrusion. This range provides a good balance between having enough volume to scatter optical radiation effectively as well as not reducing the intensity of the optical radiation too much.

In some embodiments, the protruding border 245 can be inclined (i.e., not perpendicular, in use, relative to the received/ coupled bodily tissue 250) such that scattered rays are more likely to be directed towards the bodily tissue 250. For example, the distance between opposite sides of the protruding border 245 can be wider at the end near the bodily tissue 250 and narrower at the end near the support structure 210, essentially forming an angular concave structure.

It should be noted, as would be understood by the skilled person, that in other embodiments, the optical detector arrangement 230 need not be on the other side of the bodily tissue 250 to the optical source arrangement 220. For instance, in some embodiments, the optical detector arrangement 230 can be adjacent to the optical source arrangement 220 (or at least on the same side of the support structure 210).

It should also be noted that an optical element (e.g. a mirror or an inlet of a waveguide such as an optical fibre, or an optical fiber bundle) may be provided in the position of the optical detector arrangement 230 (e.g. in the position of the optical detector arrangement 230depicted in Fig. 3) in order to detect the optical radiation. Similarly, the optical source arrangement may alternatively be provided by an optical fiber (bundle) that is likewise at least partially surrounded by a volume scattering material.

Referring now to Fig. 4, there is depicted a more detailed illustration of a cross-section of part of a PPG sensor system 300 for measuring oxygen saturation of blood within bodily tissue 350 according to a proposed embodiment.

In this embodiment, there is depicted (part of) the support structure 310, the optical source arrangement 320, the optical scatterer 340 and the received/ coupled bodily tissue 350. The other side of the support structure 310 and the optical detector arrangement 330 are not depicted (but would still be present in reality).

In this embodiment, the optical scatterer 340 fully/totally surrounds the optical source arrangement 320. All optical radiation emitted from the optical scatterer 340 is thus scattered, though as can be seen, the thickness of the optical scatterer can vary, such that optical radiation directed towards a predetermined target region and/or optical detector arrangement (such as optical radiation ray 360b) will be scattered less than shunt optical radiation directed sideways (such as optical radiation ray 360a).

As can be seen by the trajectory of optical radiation ray 360b, the optical scatterer 340 can also allow for optical radiation which reaches the bodily tissue 350 but then scatters back from the skin/tissue to potentially be scattered back to the skin and get another chance to penetrate the tissue and become functional optical radiation contributing to the PPG measurement. It is important to note that rays such as 360b, which could have been reflected by a specular or diffuse scatterer on the same spot will, with the present invention, instead be transferred to another spot to penetrate the tissue.

As can be seen by the trajectory of optical radiation ray 360c, the optical scatterer 340 can also allow for optical radiation which has been (Fresnel-)reflected back into the optical scatterer 340 at the interface between the optical scatterer 340 and the skin, to be returned to the skin, thereby giving it another chance to penetrate the tissue and become functional optical radiation contributing to the PPG measurement.

In some embodiments, the optical scatterer 340 can itself by surrounded by a low-reflective material, for instance, an absorptive material such as a black cushion. The black cushion may be formed from a black material, such as a polymer, e.g. a foam, or an elastomer that has a dark colour.

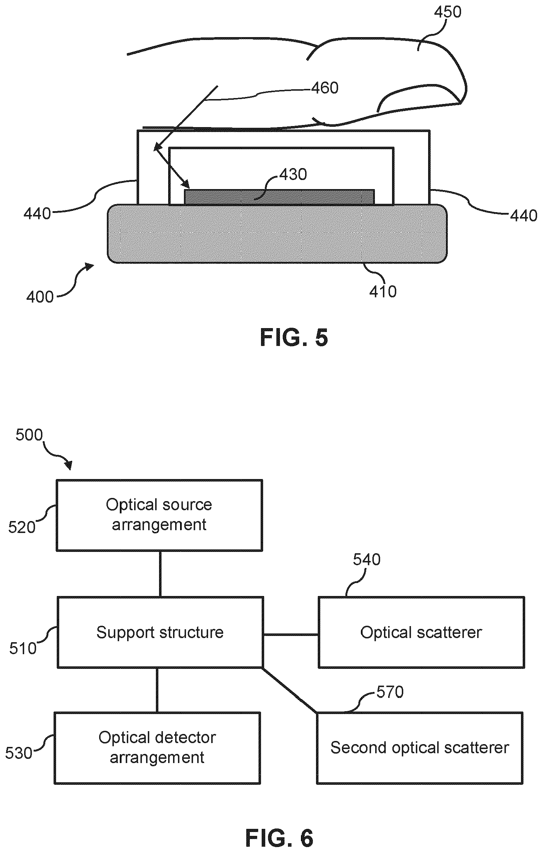

Referring now to Fig. 5, there is depicted a more detailed illustration of a cross-section of part of a PPG sensor system 400 for measuring oxygen saturation of blood within bodily tissue 450 according to a proposed embodiment.

In this embodiment, there is depicted (part of) the support structure 410, the optical detector arrangement 430, the optical scatterer 440 and the received/ coupled bodily tissue 450. The other side of the support structure 410 and the optical source arrangement 420 are not depicted (but would still be present in reality).

In this embodiment, the optical scatterer 440 at least partially (and in this case, fully) surrounds the optical detector arrangement 430. As previously explained, this can allow optical radiation which has travelled through pulsatile tissue (in bodily tissue 450), such as optical radiation ray 460, but which is directed away from the optical detector 430 to potentially be scattered towards the optical detector, thereby still helping to increase the amount of functional optical radiation which is detected and thus resulting in more accurate/reliable PPG measurements. As the skilled person would understand, in some embodiments, a (second) absorptive shell can partially surround the optical scatterer 440 which (at least partially) surrounds the optical detector arrangement 430, such that ambient optical radiation can be prevented from reaching the optical detector arrangement 430.

It should be noted that in some embodiments the optical source arrangement and/or the optical detector arrangement may be provided in the form of a lead frame device. A lead-frame LED emits rays laterally with respect to the tissue surface. With only an absorbing material surrounding the lead-frame LED, these laterally-emitted rays would be lost. With only a reflector/surface scatterer, they would be reflected into the LED housing and absorbed by the LED components (sub-mount, chip, wires). With the volume scatterer, these rays are scattered towards skin tissue instead. Similarly, an optical detector in the form of a lead-frame device captures rays arriving from a lateral direction which would otherwise be absorbed by the surrounding material and/or by the sub-mount of the optical detector.

Referring now to Fig. 6, there is depicted a simplified block diagram of a PPG sensor system 500 for measuring oxygen saturation of blood within bodily tissue according to a proposed embodiment.

The support structure 510, optical source arrangement 520, optical detector arrangement 530 and optical scatterer 540 are substantially the same as those described in relation to system 100 of Fig. 2.

This system 500, however, further includes a second optical scatterer 570. The second optical scatterer 570 is coupled to the support structure 510 and comprises a second volume scattering material; wherein, in use, the second optical scatterer 540 is configured to scatter at least a portion of the optical radiation after it has been transmitted through the received/ coupled bodily tissue 550, and preferably, the second optical scatterer 540 at least partially surrounds the optical detector arrangement 530. This further increases the amount of non-functional optical radiation potentially converted into functional optical radiation, as optical radiation which has travelled through pulsatile tissue but which is directed away from the optical detector 530 (and thus will not contribute to the PPG measurement) can be scattered towards the optical detector 530. For instance, in some embodiments, the optical scatterer 440 depicted in Fig. 5 may be the second optical scatterer. In other embodiments, such as those where the optical detector is on the same side of the received/ coupled tissue 550 as the optical radiation source, this second optical scatterer 540 may at least partially surround a mirror (configured to reflect optical radiation back towards the optical detector) or the inlet of a optical radiation guide (configured to transmit optical radiation along the optical radiation guide and out of an outlet of the optical radiation guide towards the optical detector).

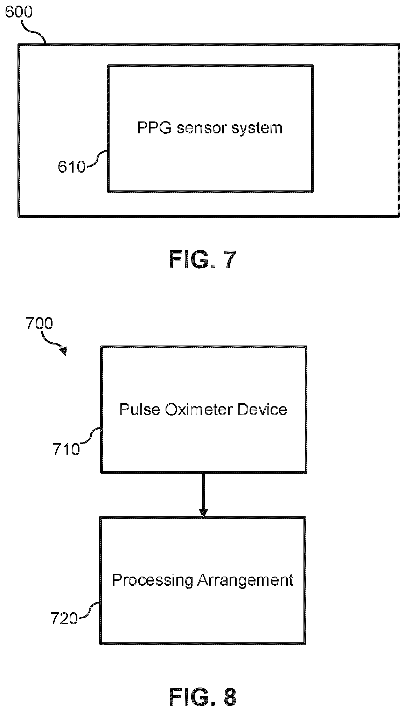

Referring now to Fig. 7, there is provided a pulse oximeter device 600. The pulse oximeter device 600 comprises the PPG sensor system 610 according to any herein-described embodiment.

For example, a pulse oximeter device 600 can comprise at least one of a finger pulse oximeter; a handheld pulse oximeter; a wrist-worn pulse oximeter; a tabletop pulse oximeter; a paediatric pulse oximeter; a foetal pulse oximeter; a phone-connected pulse oximeter; a wearable fitness tracker with pulse oximeter; an ear clip oximeter; a reflectance pulse oximeter; and a veterinary pulse oximeter.

Referring now to Fig. 8, there is depicted a simplified block diagram of a system 700 for measuring oxygen saturation of blood within bodily tissue according to a proposed embodiment.

The system 700 comprises the pulse oximetry device 710 of any herein-described embodiment (i.e., as described in relation to Fig. 7); and a processing arrangement 720. The processing arrangement 720 is configured to: receive from the pulse oximeter an optical radiation detection signal describing optical radiation detected by the optical detector arrangement of the pulse oximeter; and process the optical radiation detection signal to determine an oxygen saturation value.

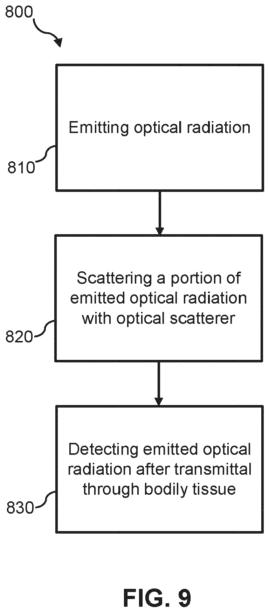

Referring now to Fig. 9, there is depicted a simplified flow diagram of a method 800 for operating a PPG sensor system according to a proposed embodiment.

The PPG sensor system comprises a support structure configured, in use, to receive or to couple to bodily tissue; an optical source arrangement coupled to the support structure and configured to emit optical radiation; an optical detector arrangement coupled to the support structure and configured to detect optical radiation incident on the optical radiation detector arrangement; and an optical scatterer coupled to the support structure and comprising a volume scattering material. For instance, the PPG sensor system can be a PPG sensor system according to any herein-described embodiment.

The method 800 comprises step 810 of emitting optical radiation from the optical source arrangement; step 820 of scattering at least a portion of the emitted optical radiation with the optical scatterer; and step 830 of detecting at least a portion of the emitted optical radiation at the optical detector arrangement after it has been transmitted through at least a part of the received/ coupled bodily tissue.

For example, in this embodiment, the at least a portion of the emitted optical radiation comprises optical radiation emitted from the optical source arrangement and directed outside a predetermined target region (e.g., as explained in relation to Fig. 3).

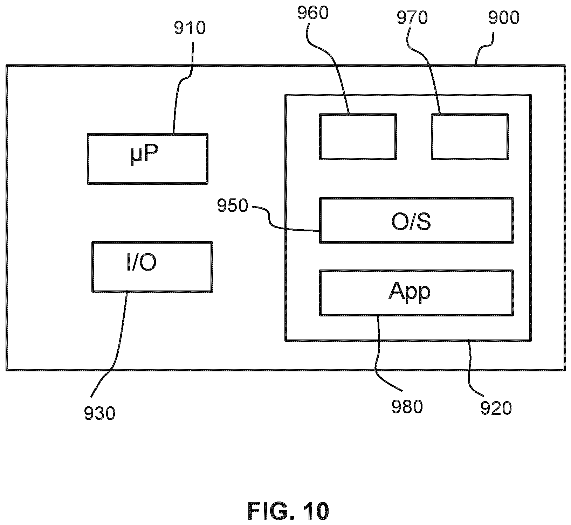

Fig. 10 illustrates an example of a computer 900 within which one or more parts of an embodiment may be employed. Various operations discussed above may utilize the capabilities of the computer 900. In this regard, it is to be understood that system functional blocks can run on a single computer or may be distributed over several computers and locations (e.g. connected via internet).

The computer 900 includes, but is not limited to, PCs, workstations, laptops, PDAs, palm devices, servers, storages, and the like. Generally, in terms of hardware architecture, the computer 900 may include one or more processors 910, memory 920 and one or more I/O devices 930 that are communicatively coupled via a local interface (not shown). The local interface can be, for example but not limited to, one or more buses or other wired or wireless connections, as is known in the art. The local interface may have additional elements, such as controllers, buffers (caches), drivers, repeaters, and receivers, to enable communications. Further, the local interface may include address, control, and/or data connections to enable appropriate communications among the aforementioned components.

The processor 910 is a hardware device for executing software that can be stored in the memory 920. The processor 910 can be virtually any custom made or commercially available processor, a central processing unit (CPU), a digital signal processor (DSP), or an auxiliary processor among several processors associated with the computer 900, and the processor 910 may be a semiconductor based microprocessor (in the form of a microchip) or a microprocessor.

The memory 920 can include any one or combination of volatile memory elements (e.g., random access memory (RAM), such as dynamic random access memory (DRAM), static random access memory (SRAM), etc.) and non-volatile memory elements (e.g., ROM, erasable programmable read only memory (EPROM), electronically erasable programmable read only memory (EEPROM), programmable read only memory (PROM), tape, compact disc read only memory (CD-ROM), disk, diskette, cartridge, cassette or the like, etc.). Moreover, the memory 920 may incorporate electronic, magnetic, optical, and/or other types of storage media. Note that the memory 920 can have a distributed architecture, where various components are situated remote from one another, but can be accessed by the processor 910.

The software in the memory 920 may include one or more separate programs, each of which comprises an ordered listing of executable instructions for implementing logical functions. The software in the memory 920 includes a suitable operating system (O/S) 950, compiler 960, source code 970, and one or more applications 980 in accordance with exemplary embodiments. As illustrated, the application 980 comprises numerous functional components for implementing the features and operations of the exemplary embodiments. The application 980 of the computer 900 may represent various applications, computational units, logic, functional units, processes, operations, virtual entities, and/or modules in accordance with exemplary embodiments, but the application 980 is not meant to be a limitation.

The operating system 950 controls the execution of other computer programs, and provides scheduling, input-output control, file and data management, memory management, and communication control and related services. It is contemplated by the inventors that the application 980 for implementing exemplary embodiments may be applicable on all commercially available operating systems.

Application 980 may be a source program, executable program (object code), script, or any other entity comprising a set of instructions to be performed. When a source program, then the program is usually translated via a compiler (such as the compiler 960), assembler, interpreter, or the like, which may or may not be included within the memory 920, so as to operate properly in connection with the O/S 950. Furthermore, the application 980 can be written as an object oriented programming language, which has classes of data and methods, or a procedure programming language, which has routines, subroutines, and/or functions, for example but not limited to, C, C++, C#, Pascal, Python, BASIC, API calls, HTML, XHTML, XML, ASP scripts, JavaScript, FORTRAN, COBOL, Perl, Java, ADA, .NET, and the like.

The I/O devices 930 may include input devices such as, for example but not limited to, a mouse, keyboard, scanner, microphone, camera, etc. Furthermore, the I/O devices 930 may also include output devices, for example but not limited to a printer, display, etc. Finally, the I/O devices 930 may further include devices that communicate both inputs and outputs, for instance but not limited to, a NIC or modulator/demodulator (for accessing remote devices, other files, devices, systems, or a network), a radio frequency (RF) or other transceiver, a telephonic interface, a bridge, a router, etc. The I/O devices 930 also include components for communicating over various networks, such as the Internet or intranet.

If the computer 900 is a PC, workstation, intelligent device or the like, the software in the memory 920 may further include a basic input output system (BIOS) (omitted for simplicity). The BIOS is a set of essential software routines that initialize and test hardware at start-up, start the O/S 950, and support the transfer of data among the hardware devices. The BIOS is stored in some type of read-only-memory, such as ROM, PROM, EPROM, EEPROM or the like, so that the BIOS can be executed when the computer 900 is activated.

When the computer 900 is in operation, the processor 910 is configured to execute software stored within the memory 920, to communicate data to and from the memory 920, and to generally control operations of the computer 900 pursuant to the software. The application 980 and the O/S 950 are read, in whole or in part, by the processor 910, perhaps buffered within the processor 910, and then executed.

When the application 980 is implemented in software it should be noted that the application 980 can be stored on virtually any computer readable medium for use by or in connection with any computer related system or method. In the context of this document, a computer readable medium may be an electronic, magnetic, optical, or other physical device or means that can contain or store a computer program for use by or in connection with a computer related system or method.

The application 980 can be embodied in any computer-readable medium for use by or in connection with an instruction execution system, apparatus, or device, such as a computer-based system, processor-containing system, or other system that can fetch the instructions from the instruction execution system, apparatus, or device and execute the instructions. In the context of this document, a "computer-readable medium" can be any means that can store, communicate, propagate, or transport the program for use by or in connection with the instruction execution system, apparatus, or device. The computer readable medium can be, for example but not limited to, an electronic, magnetic, optical, electromagnetic, infrared, or semiconductor system, apparatus, device, or propagation medium.

The systems of Figs. 1-5 and 7, the device of Fig. 7 and the method of Fig. 9, may be implemented in hardware or software, or a mixture of both (for example, as firmware running on a hardware device). To the extent that an embodiment is implemented partly or wholly in software, the functional steps illustrated in the process flowcharts may be performed by suitably programmed physical computing devices, such as one or more central processing units (CPUs) or graphics processing units (GPUs). Each process - and its individual component steps as illustrated in the flowcharts - may be performed by the same or different computing devices. According to embodiments, a computer-readable storage medium stores a computer program comprising computer program code configured to cause one or more physical computing devices to carry out an encoding or decoding method as described above when the program is run on the one or more physical computing devices.

Storage media may include volatile and non-volatile computer memory such as RAM, PROM, EPROM, and EEPROM, optical discs (like CD, DVD, BD), magnetic storage media (like hard discs and tapes). Various storage media may be fixed within a computing device or may be transportable, such that the one or more programs stored thereon can be loaded into a processor.

To the extent that an embodiment is implemented partly or wholly in hardware, the blocks shown in the block diagrams of Fig. 7 may be separate physical components, or logical subdivisions of single physical components, or may be all implemented in an integrated manner in one physical component. The functions of one block shown in the drawings may be divided between multiple components in an implementation, or the functions of multiple blocks shown in the drawings may be combined in single components in an implementation. Hardware components suitable for use in embodiments of the present invention include, but are not limited to, conventional microprocessors, application specific integrated circuits (ASICs), and field-programmable gate arrays (FPGAs). One or more blocks may be implemented as a combination of dedicated hardware to perform some functions and one or more programmed microprocessors and associated circuitry to perform other functions.

A single processor or other unit may fulfil the functions of several items recited in the claims. The mere fact that certain measures are recited in mutually different dependent claims does not indicate that a combination of these measures cannot be used to advantage. If a computer program is discussed above, it may be stored/distributed on a suitable medium, such as an optical storage medium or a solid-state medium supplied together with or as part of other hardware, but may also be distributed in other forms, such as via the Internet or other wired or wireless telecommunication systems. If the term "adapted to" is used in the claims or description, it is noted the term "adapted to" is intended to be equivalent to the term "configured to". Any reference signs in the claims should not be construed as limiting the scope.

The flowchart and block diagrams in the Figures illustrate the architecture, functionality, and operation of possible implementations of systems, methods, and computer program products according to various embodiments of the present invention. In this regard, each block in the flowchart or block diagrams may represent a module, segment, or portion of instructions, which comprises one or more executable instructions for implementing the specified logical function(s). In some alternative implementations, the functions noted in the block may occur out of the order noted in the figures. For example, two blocks shown in succession may, in fact, be executed substantially concurrently, or the blocks may sometimes be executed in the reverse order, depending upon the functionality involved. It will also be noted that each block of the block diagrams and/or flowchart illustration, and combinations of blocks in the block diagrams and/or flowchart illustration, can be implemented by special purpose hardware-based systems that perform the specified functions or acts or carry out combinations of special purpose hardware and computer instructions, the architecture, functionality, and operation of possible implementations of systems, methods, and computer program products according to various embodiments of the present invention.

## Claims

1. A PPG sensor system (100) for measuring oxygen saturation of blood within bodily tissue, the system comprising:
a support structure (110) configured, in use, to receive or to couple to bodily tissue;
an optical source arrangement (120) coupled to the support structure and configured to emit optical radiation;
an optical detector arrangement (130) coupled to the support structure and configured to detect optical radiation incident on the optical detector arrangement; and
an optical scatterer (140) coupled to the support structure and comprising a volume scattering material;
wherein, in use, the optical scatterer is configured to scatter at least a portion of the emitted optical radiation.

2. The PPG sensor system of claim 1, wherein the at least a portion of the emitted optical radiation comprises optical radiation emitted from the optical source arrangement and directed outside of a predetermined target region (225).

3. The PPG sensor of claim 2, wherein the optical scatterer (240) is configured such that, in use, optical radiation emitted from the optical source arrangement (220) and directed towards the predetermined target region (225) is transmitted directly into the received or the coupled bodily tissue (250) without being scattered by the optical scatterer.

4. The PPG sensor of claim 3, wherein the optical scatter (140) is laterally offset relative to the optical source arrangement (120).

5. The PPG sensor system of any of claims 2 to 4, wherein the optical scatterer (140) at least partially surrounds the optical source arrangement (120).

6. The PPG sensor of claim 5, wherein the optical scatterer (240) comprises an opening for directly transmitting optical radiation emitted from the optical source arrangement (220) and directed towards the predetermined target region (225) without being scattered by the optical scatterer.

7. The PPG sensor system of claim 6, wherein the optical scatterer (240) comprises a protruding border (245) surrounding the opening, protruding, in use, towards the received or the coupled bodily tissue (250).

8. The PPG sensor system of claim 7, wherein the protruding border (245) is 0.5 to 5 millimetres thick.

9. The PPG sensor of any of claims 6-8, wherein the opening has a similar, or the same, footprint as the optical source arrangement.

10. The PPG sensor system of any preceding claim, wherein the volume scattering material comprises a polymer or a ceramic having scattering particles and/or voids embedded therein.

11. The PPG sensor system of any preceding claim, wherein the system further comprises a second optical scatterer (570) coupled to the support structure (510) and comprising a second volume scattering material; wherein, in use, the second optical scatterer is configured to scatter at least a portion of the optical radiation after it has been transmitted through the received or the coupled bodily tissue, and optionally wherein the second optical scatterer at least partially surrounds the optical detector arrangement (530).

12. The PPG sensor system of claim 1, wherein the optical scatterer (440) at least partially surrounds the optical detector arrangement (430).

13. A pulse oximeter device (600) comprising the PPG sensor system (610) of any of claims 1 to 12.

14. A system (700) for measuring oxygen saturation of blood within bodily tissue, the system comprising:
the pulse oximeter device (710) of claim 13; and
a processing arrangement (720) configured to:
receive from the pulse oximeter an optical radiation detection signal describing optical radiation detected by the optical detector arrangement of the pulse oximeter; and
process the optical radiation detection signal to determine an oxygen saturation value.

15. A method (800) for operating a PPG sensor system, the PPG sensor system comprising:
a support structure configured, in use, to receive or to couple to bodily tissue;
an optical source arrangement coupled to the support structure and configured to emit optical radiation;
an optical detector arrangement coupled to the support structure and configured to detect optical radiation incident on the optical radiation detector arrangement; and
an optical scatterer coupled to the support structure and comprising a volume scattering material; and
the method comprising:
emitting optical radiation (810) from the optical source arrangement; scattering at least a portion of the emitted optical radiation (820) with the optical scatterer; and
detecting at least a portion of the emitted optical radiation (830) at the optical detector arrangement after it has been transmitted through at least a part of the received or the coupled bodily tissue.

16. The method of claim 15, wherein the at least a portion of the emitted optical radiation comprises optical radiation emitted from the optical source arrangement and directed outside of a predetermined target region.
